# EUROPEAN PATENT SPECIFICATION

(11) **EP 1 708 693 B1**
(45) Date of publication and mention of the grant of the patent: **04.03.2009**
(21) Application number: 04803751.9
(22) Date of filing: 10.12.2004
(51) Int. Cl.: A61K 31/282, A61P 35/00

(54) **PHARMACEUTICAL COMPOSITIONS CONTAINING PALTINUM COMPLEXES WITH SECONDARY XANTHATES AND THERAPEUTIC USES THEREOF**
PHARMAZEUTISCHE ZUSAMMENSETZUNGEN MIT PLATIN-KOMPLEXEN MIT SEKUNDÄREN XANTHATEN UND IHRE THERAPEUTISCHEN ANWENDUNGEN
COMPOSITION PHARMACEUTIQUE COMPRENANT UN COMPLEXE DE PLATINE AVEC DEX XANTHATES SECONDAIRES ET SON UTILISATION THERAPEUTIQUE

(30) Priority: 10.12.2003 EP 03028298
(43) Date of publication of application: 11.10.2006
(73) Proprietor: Deutsches Krebsforschungszentrum Stiftung des öffentlichen Rechts, 69120 Heidelberg (DE); Ruprecht-Karls-Universität Heidelberg, 69117 Heidelberg (DE)
(72) Inventor: AMTMANN, Eberhard, 69121 Heidelberg (DE); FRIEBOLIN, Wolfgang, 69123 Heidelberg (DE); SCHILLING, Gerhard, 68526 Ladenburg (DE)
(74) Representative: Schüssler, Andrea
(86) International application number: PCT/EP2004/014110
(87) International publication number: WO 2005/058307

(56) References cited:
- US-A1- 2002 004 526
- OSA T ET AL: "SYNTHESIS AND ANTITUMOR ACTIVITY OF CIS-DICHLOROPLATINUM COMPLEXES COORDINATING NITROGEN CYCLIC OR SULFUR COMPOUNDS" CHEMICAL AND PHARMACEUTICAL BULLETIN, PHARMACEUTICAL SOCIETY OF JAPAN. TOKYO, JP, vol. 34, no. 9, 1986, pages 3563-3572, XP000907123 ISSN: 0009-2363
- CRACIUNESCU ET AL: "Dual in vivo pharmacological activities (antitrypanosomal and antitumor ) of some new complexes of platinum(II) and platinum(IV)" ANALES DE LA REAL ACADEMIA DE FARMACIA, XX, XX, vol. 58, no. 4, 1992, pages 529-549, XP000910520 ISSN: 0034-0618

## Description

The present invention relates to pharmaceutical compositions containing certain platinum complexes with secondary xanthates and to the use thereof for treating tumors and autoimmune diseases.

Chemotherapy is still indispensable for the treatment of cancer. Despite advances in surgery and radiotherapy, mortality remained virtually unchanged for most cancers in the decades before discovering cis-platin [cis-dichloro-diammine-platinum(II)], which was a major step forward in the treatment of cancers of, e.g,. ovary, lung and testis. Today platinum compounds remain an important component of chemotherapeutic regimens. All platinum compounds in clinical use are derivatives of cisplatin with two amino groups in the *cis-*position.

Yet, a perfect chemotherapeutic agent is still lacking. Recently a platinum complex based on sulfur as complex forming atoms, bis(O-ethyl-dithiocarbonato)platinum(II), named "thioplatin" with anti-tumoral activity against a number of human tumor lines was described (Amtmann et al., Cancer Chemother. Pharmacol. 47 (2001), 461-466; US 2002/004526; EP-B1-1 107 751). Surprisingly, it was found that Thioplatin displayed significantly higher cytotoxicity when tumor cells were cultivated in media of pH 6.8 as compared to media of pH 7.4. Since in solid tumors a pH of 6.8 and lower has been frequently observed an improved therapeutic index with Thioplatin could be expected. Indeed, Thioplatin displayed anti-tumoral activity on human tumors xenotransplanted in nude mice which was comparable to cisplatin, yet a significantly lower toxicity on kidneys, small intestines and on white blood cell count was encountered. However, it has been discovered that "thioplatin" is not sufficiently soluble, and, thus, there is a need for complexes of platinum and xanthogenate showing improved solubulity and even higher cytostatic activity and, preferably, lower side effects.

Thus, it is the object of the present invention to provide pharmaceutical compositions containing complexes of platinum and xanthogenate having a higher cytostatic activity, i.e., are more effective in low dosage and have a lower toxic effect on healthy cells, compared to the complexes available so far.

The solution to said technical problem is achieved by providing the embodiments characterized in the claims.

In order to establish structure-activity relationships, derivatives of "thioplatin" (= Bis(O-ethyldithiocarbonato)platinum(II)) were prepared and analyzed. Twenty different bis(O-alkyl-dithiocarbonato)platinum(II)-complexes were synthesized and tested for cytotoxic activity in a panel of six human tumor lines. Derivatives with up to 7-fold increased activity as compared to "thioplatin" and up to 25-fold more activity than cis-platin could be characterized. When various bis(O-dithiocarbonato)platinum(II)-complexes were analyzed for anti-tumoural activity in vitro, a correlation between lipophilicity of residues and activity was found. Complexes derived from secondary xanthates displayed significantly higher activity than those derived from primary xanthates with the same number of C atoms. Like "thioplatin", all tested platinum complexes were more active at pH 6.8 than at pH 7.4. A pH of 6.8 and lower has been frequently found in solid tumors, due to the tendency of tumor cells to anaerobic fermentation. Drugs with such pH-dependent anti-tumoral activity have an improved therapeutic index as compared to drugs which are active irrespectively from pH.

Thus, the present invention relates to a pharmaceutical composition containing at least one of the following (active) compounds: Bis(O-isopropyldithiocarbonato)platinum(II), Bis(O-(1-ethyl)propyldithiocarbonato)platinum(II), Bis(O-(1,2,2-trimethyl)propyldithiocarbonato)platinum(II), Bis(O-cyclobutyldithiocarbonato)platinum(II), Bis(O-cyclopentyldithiocarbonato)platinum(II) and/or Bis(O-cyclohexyldithiocarbonato)platinum(II).

The expression "at least one" means one, two, three or more, up to all of the listed compounds.

The above mentioned compounds are preferably produced by a process which is characterized in that a ligand exchange reaction from a platinum complex, such as e.g. cis-chlorodiammineplatinum (II), is carried out with the corresponding xanthogenate in known manner. Such a process is, e.g.; described in the examples below and EP-Bl-1 107 751.

The compounds of the present invention are suitable for treating various cancerous diseases, such as testicular tumors, ovarian carcinomas, bladder carcinomas, colonic prostatic carcinomas, parvocellular and non-parvocellular bronchial carcinomas, carcinomas of the cephalic and cervical parts, carcinomas of the thoracic and abdominal regions, cervical and endometrial carcinomas, sarcomas and melanomas as well as leukemias. The treatment of the parvocellular bronchial carcinoma or colorectal carcinoma is preferred. The treatment can also be carried out as a treatment associated with a radiotherapy or before and/or after an operation.

In addition, the compounds of the present invention are suitable for treating an autoimmune disease, e.g., rheumatic arthritis, lupus erythematodes, Crohn's disease, colitis ulcerosa, multiple sclerosis.

The compounds of the present invention are well tolerable and show an up to 7-fold increased activity as to compared to "thioplatin" and up to 25-fold increased activity as compared to cis-platin. When a dosage having good antitumoral effects is used, hardly any side-effects occur. In particular, the feared nephrotoxicity known for cis-platin has not yet occurred in this way in the case of these compounds. Another advantage of the compounds according to the invention is that they have a broad activity spectrum against the most varying tumors and are particularly also effective against tumors which have resisted treatment with platinum compounds (e.g. cis-platin) so far. The compounds of the invention are particularly suitable for solid tumors.

Another advantage consists in that the effectiveness of the compounds according to the invention is greater in the slightly acidic pH range than in the alkaline one, since many tumor tissues have a rather acidic environment.

The pharmaceutical composition according to the invention can be administered in various ways, e.g. orally, parenterally, cutaneously, subcutaneously, intravenously, intramuscularly, rectally or intratuomorally. The intravenous or intratumoral administration is preferred, i.e. the administration in certain diseased organs or parts of the body. The pharmaceutical composition is administered to a patient over a period to be determined by a physician.

The dosage of the compound according to the invention is determined by a physician by means of the patient-specific parameters, such as age, weight, sex, severity of the disease, etc. The dosage is preferably from 0.001 to 1000 mg/kg body weight.

In accordance with the kind of administration, the pharmaceutical composition is formulated in a suitable way, e.g. in the form of simple or coated tablets, hard or soft gelatin capsules, powders for reconstitution prior to use, granular powders, suppositories, ovules, injectables, infusion solutions, pomades, creams, gels, microspheres, implants, which are produced according to conventional galenic processes.

The compounds of the present can optionally be administered together with further active substances and with excipients common in pharmaceutical compositions, e.g. depending on the preparation to be produced talcum, gum arabic, lactose, starch, magnesium stearate, cocoa butter, aqueous and nonaqueous carriers, adipoids of animal or vegetable origin, paraffin derivatives, glycols (particularly polyethylene glycol), various plasticizers, dispersants or emulsifiers, preservatives.

Additives such as sodium chloride solution, ethanol, sorbitol, glycerol, olive oil, almond oil, propylene glycol or ethylene glycol can be used for the production of liquid preparations.

Infusions or injectable solutions are preferably produced. They are preferably aqueous solutions or suspensions, it being possible to produce them prior to use, e.g. from lyophilized preparations containing the active substance as such or together with a carrier such as mannitol, lactose, glucose, albumin and the like. The ready-to-use solutions are sterilized and optionally mixed with adjuvants, e.g. preservatives, stabilizers, emulsifiers, solution aids, buffers and/or salts for controlling the osmotic pressure. Sterilization can be achieved by sterile filtration through filters having a small pore size, whereupon the composition can optionally be lyophilized. Small amounts of antibiotics can also be added so as to maintain sterility.

The provision of the pharmaceutical preparation according to the invention in a unit dosage form for administration to a mammal requiring anticancer treatment is advantageous.

The invention also relates to pharmaceutical compositions which contain a therapeutically effective amount of the active ingredient (compound according to the invention) together with organic or inorganic inert solid or liquid pharmaceutically compatible carriers and diluents, respectively, which are suited for the intended administration and which show no unfavorable interactions with the active ingredients.

The pharmaceutical compositions according to the invention comprise as active substance at least one active substance as defined above. Optionally further pharmaceutical active substances can be added to the composition, such as immunosuppressive agents, e.g. cyclosporine, rapamycin, 15-deoxyspergualine, OKT3, azathioprine; cytokines (e.g. TNF), interferon, etc. In addition, the composition according to the invention can additionally contain a steroid or further cytostatic agents (e.g., methotrexate, aminopterin, dacarbacine, nitroso urea compounds, fluorouracil, bleomycin, daunomycin, daunorubicin, doxorubicin, mithramycin, mitomycin C, etc.).

The invention also relates to a process for the production of a pharmaceutical composition, which is characterized by mixing a compound of the invention with a pharmaceutically compatible carrier.

### Brief description of the drawings

Fig. 1: Cytotoxic activity on human cancer cell lines IC₅₀-values for cytotoxicity in MCF-7, Calu-6, LXF-289, SK-MEL-25, SK-OV3 and KB cells at pH 6.8. Bars in the background indicate IC₅₀-values obtained in the corresponding cell line at pH 7.4 each. Exact values and SD are indicated in Table 1.
Fig. 2: Mean quotient of IC₅₀ at pH 7.4/IC₅₀ at pH 6.8 Mean values ± SD of IC₅₀ at pH 7.4/ IC₅₀ at pH 6.8 for all six human tumor cell lines tested. IC₅₀at both pH conditions were obtained for four cell lines.

The following examples illustrate the invention.

### Example 1

### Synthesis of bis(O-alkyl-dithiocarbonato)platinum(II)-complexes 15-20

All alkylxanthates were prepared according to published procedures (Jensen et al., Acta Chem. Scand. 1969, 23, 1916-1934; Rao S. R.; "Xanthates and Related Compounds", 1971, Marcel Dekker, New York). Xanthates were purified by crystallization or precipitation from acetone-diethylether or pentane. Colourless to bright yellow crystals or solids were obtained. Potassium (or sodium) alkylxanthates (KS₂COR or NaS₂COR with R = corresponding to compounds 15-20) were reacted with dipotassium tetrachloroplatinate(II) (K₂PtCl₄) as illustrated in the following scheme:

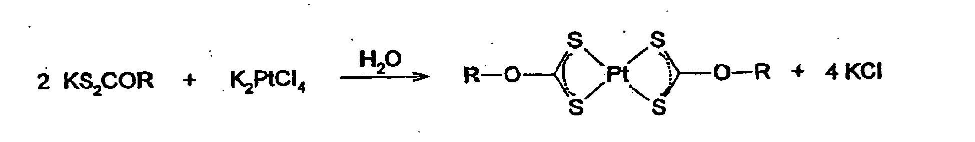

A solution of 3.6 mmol of the suitable potassium alkylxanthate in 5 ml water was added to 1.2 mmol K₂PtCl₄ dissolved in 10 ml water. Immediate precipitation of a yellow solid could be observed. In the cases of alkylxanthates with long carbon chains the reaction progressed only slowly. The mixture was stirred overnight at room temperature. The precipitate was filtered, washed three times with distilled water and was crystallized from acetone/chloroform. Remaining solvent was removed *in vacuo* at 10⁻³ torr for 1-2 days. The isolated yields ranged between 73-99%.

Structures of bis(O-alkyl-dithiocarbonato)platinum(II)-complexes were verified by ¹H, ¹³C and ¹⁹⁵Pt NMR-, IR- and UV-spectroscopy and by mass spectrometry. Combustion analysis (C, H, S) agreed with the calculated values within a range of ± 0.3 %.

All platinum(II)-complexes formed yellow crystals, which were stable at room temperature in the presence of light and air for several weeks. They were insoluble in water, slightly soluble in DMSO or acetone (1-3 mg/ml) and soluble in CHCl₃. All derivatives were crystallized from acetone or acetone/CHCl₃ solutions. While being rather unstable in DMSO, the complexes proved to be stable when dissolved in CHCl₃ or acetone over a period of 1-2 month, when stored at 4 °C.

Melting points were determined in open capillaries with a Büchi electrothermal melting point apparatus and are not corrected. ¹H and ¹³C NMR spectra were recorded in CDCl₃ on a Brucker AC 300 instrument operating at 300 and 75 MHz respectively. Chemical shifts (δ) are given in parts per million (ppm) relative to tetramethylsilane as internal reference. The following abbreviations are used to describe peak pattern when appropriate: s (singlet), d (dublet), t (triplet), q (quartet), p (pentet), m (multiplet), c (centered). ¹⁹⁵Pt NMR spectra were recorded in CDCl₃ on a Brucker DRX 300 instrument operating at 107 MHz with K₂PtCl₄ as internal reference. Infrared Spectra were recorded on a Brucker Vector 22 in KBr. The following abbreviations are used to describe the peak intensity: w (weak), m (medium), s (strong). UV spectra were recorded on a Hewlett Packard HP 8452 A spectrophotometer in CH₂Cl₂ as solvent. The absorption maxima λₘₐₓ are reported in nm, the extinction coefficient ε in dm-³mol⁻¹cm⁻¹. Mass spectra were obtained with a JEOL JMS-700 (FAB, positive mode, matrix was m-nitrobenzylalcohole). Cobustion analysis (C, H, S) was performed on an Elementar vario EL and the results were within ±0.3% of the theoretical values. All commercially available chemicals were used as received (solvents in p.a. quality).
**1. Bis(O-isopropyl-dithiocarbonato)platinum(II) (15):** yield 93%; mp 159°C; ¹H NMR δ 1.52 (d, ³*J* = 6.2 Hz, 6H, CH₃) , 5.44 (sept. , ³*J* = 6.2 Hz, 1H, OCH) ; ¹³C NMR δ 21.61 (2xCH₃) , 79.08 (OCH), 233.95 (CS₂); ¹⁹⁵Pt NMR δ -4190; IR (KBr) 2980 (w), 2929 (w), 1627 (w), 1446 (w), 1362 (s), 1287 (s), 1082 (s), 1007 (m), 897 (w); MS (FAB+) m/z 154, 289, 307, 380, 381, 422, 421, 423, 464, 465 (M⁺), 466, 467; UV [nm(ε)] λ 252 (29500), 304 (4700), 358 (5700), 448 (2800); Anal. (C₈H₁₄O₂S₄Pt) C, H, S.
**2. Bis(O-(1-ethyl)propyl-dithiocarbonato)platinum(II) (16):** yield 90%; mp 92°C; ¹H NMR δ 0.97 (t, ³*J*= 7.4 Hz, 12H, CH₃) , 1.78-1.86 (m, 8H, CH₂), 5.12 (m_{c}, 2H, OCH). ¹³C NMR δ 9.50 (2xCH₃), 26.51 (2xCH₂), 88.89 (OCH), 234.79 (CS₂); ¹⁹⁵Pt NMR δ - 4190; IR (KBr) 2968 (w), 2935 (w), 1638 (w), 1457 (w), 1385 (m), 1370 (m), 1304 (s), 1287 (s), 1103 (w), 1017 (m); MS (FAB+) m/z 380, 381, 450, 451, 452, 520, 521 (M⁺), 522, 523, 1042 (M⁺_{Dimer}), 1043 ; UV [nm(ε)] λ 252 (44600), 304 (6400), 358 (7300), 446 (2300) ; Anal. (C₁₂H₂₂O₂S₄Pt) C, H, S.
**3. Bis(O-(1,2,2-trimethyl)propyl-dithiocarbonato)platinum(II) (17):** yield 88%; mp 170°C (decomp.); ¹H NMR δ 0.98 (s, 18H, CH₃) , 1.38 (d, ³*J* = 6.4 Hz, 6H, CH₃), 5.05 (q, ³*J* = 6.4 Hz, 2H, CH); ¹³C NMR δ 14.80 (CH₃) , 25.56 ((CH₃)₃) , 34.99 (Cq), 89.61 (OCH), 234.46 (CS₂); ¹⁹⁵Pt NMR δ -4195; IR (KBr) 2964 (m), 2870 (w), 1628 (w), 1441 (w), 1370 (s), 1291 (s), 1118(w), 1075 (m), 1045 (m), 1015 (m), 873 (m); MS (FAB+) m/z 307, 381, 405, 466, 547, 548, 549, 550 (M⁺), 551, 552, 553, 668, 1099 (M⁺_{Dimer}) ; UV [nm(ε)] λ 252 (38800), 304 (5700), 358 (6800), 446 (2400); Anal. (C₁₄H₂₆O₂S₄Pt) C, H, S.
**4. Bis(O-cyclobutyl-dithiocarbonato)platinum(II) (18):** yield 79%; mp 155°C; ¹H NMR δ 1.71 (m_{c}, 2H, CH₂), 1.93 (m_{c}, 2H, CH₂), 2.37-2.44 (m, 4H, CH₂), 2.45-2.57 (m, 4H, CH₂), 5.35 (p, ³*J* = 6.4 Hz, 2H, OCH) ; ¹³C NMR δ 13.55 (CH₂) , 30.10 (2xCH₂), 77.23 (OCH) , 233.31 (CS₂); ¹⁹⁵Pt NMR δ -4210; IR (KBr) 2990 (w), 2942 (w), 1637 (w), 1364 (m), 1278 (s), 1261 (s), 1126 (m), 1126 (m), 1040 (m), 1014 (m), 885 (m) ; MS (FAB+) m/z 307, 460, 489, 490 (M⁺), 491, 492, 979, 980 (M⁺_{Dimer}) ; UV [nm(ε)] λ 252 (45700), 306 (6900), 362 (7700), 450 (3100); Anal. (C₁₀H₁₄O₂S₄Pt) C, H, S.
**5. Bis(O-cyclopentyl-dithiocarbonato)platinum(II) (19):** yield 75%; mp 156°C (decomp.); ¹H NMR δ 1.63-1.71.(m, 4H, CH₂), 1.72-1.86 (m, 4H, CH₂), 1.96-2.10 (m, 8H, CH₂), 5.58 (m, 2H, OCH); ¹³C NMR δ 23.80 (2xCH₂), 32.81 (2xCH₂), 88.02 (OCH), 233.95 (CS₂); ¹⁹⁵Pt NMR δ -4196; IR (KBr) 2960 (w), 2924 (w), 1637 (w), 1429 (w), 1369 (m), 1331 (m), 1282 (s), 1156 (m), 1020 (m), 925 (m) ; MS (FAB+) m/z 307, 329, 460, 517, 518 (M⁺) , 519; UV [nm(c)]: λ 252 (39400), 304 (5400), 360 (6300), 448 (1900) ; Anal. (C₁₂H₁₈O₂S₄Pt) C, H, S.
**6. Bis(O-cyclohexyl-dithiocarbonato)platinum(II) (20):** yield 97%; mp 162°C (decomp.); ¹H NMR δ 1.28-1.85 (m, 16H, CH₂), 1.95-2.07 (m, 4H, CH₂), 5.15 (m, 2H, OCH); ¹³C NMR δ 23.30 (CH₂), 24.87 (CH₂), 31.14 CH₂), 83 .66 (OCH), 233.84 (CS₂) ; ¹⁹⁵Pt NMR δ -4189; IR (KBr) 2935 (m), 2856 (m), 1626 (w), 1448 (m), 1377 (m), 1281 (s), 1241 (m), 1151 (m), 1033 (m), 1018 (s), 993 (m), 907 (m), 882 (m); MS (FAB+) m/z 460, 482, 544, 545, 546 (M⁺) , 547, 548; UV [nm(ε)]λ 252 (36800), 304 (5800), 360 (7200), 448 (3200) ; Anal. (C₁₄H₂₂O₂S₄Pt) C, H, S. Crystal system: Monoclinic; space group P2₁/c; with a = 10.3154(4) Å, b = 9.4968(3) Å, c = 18.6967(7) Å and α = 90°, β = 100.118(1) °, γ = 90°. Distances: Pt1-S2 2.3182(7) Å, Pt1-S1-2.3200(7) Å, Pt1-S1#1 2.3237(6) Å, Angles: S1-Pt1-S1#1 177.19(3)°, S1-Pt1-S2#1 104.67(2)°, S1-Pt1-S2 74.94(2)°.

### Example 2

### Determination of the antitumoral activity of compounds 15 to 20 against human cancer cells in tissue culture

### (A) Cell lines and materials

Calu-6 (adenocarcinoma, lung), SK-MEL-25 (melanoma), MCF-7 (mamma carcinoma), LXF-289 (adenocarcinoma, lung), SK-OV -3 (adenocarcinoma, ovary), KB (squameous cell carcinoma, head and neck) were obtained from ATCC and were cultivated according to the instructions of the supplier at 37°C, 5% CO₂ atmosphere and 100% relative humidity.

PtCl₂(NH₃)₂, was purchased from Bristol Myers as a solution of 0.5 mg/ml (Platinex). Thioplatin was obtained from Antisoma, London.

### (B) Cytotoxicity and structure-activity relationship

The anti-tumoral activity of compounds was determined in tissue culture media of pH of 6.8 and 7.4. Cells were incubated for 2 h with the test compounds, followed by 24 - 96 h incubation with regular, inhibitor free medium of pH 7.4. The substances were serially diluted in tissue culture medium of the desired pH. After incubation, surviving cells were stained with crystal violet and the optical density was measured in an ELISA reader. The concentration which was needed to reduce the cell number by 50 % (IC₅₀) was determined from dose-response curves for each compound. Results are enlisted in Table 1.

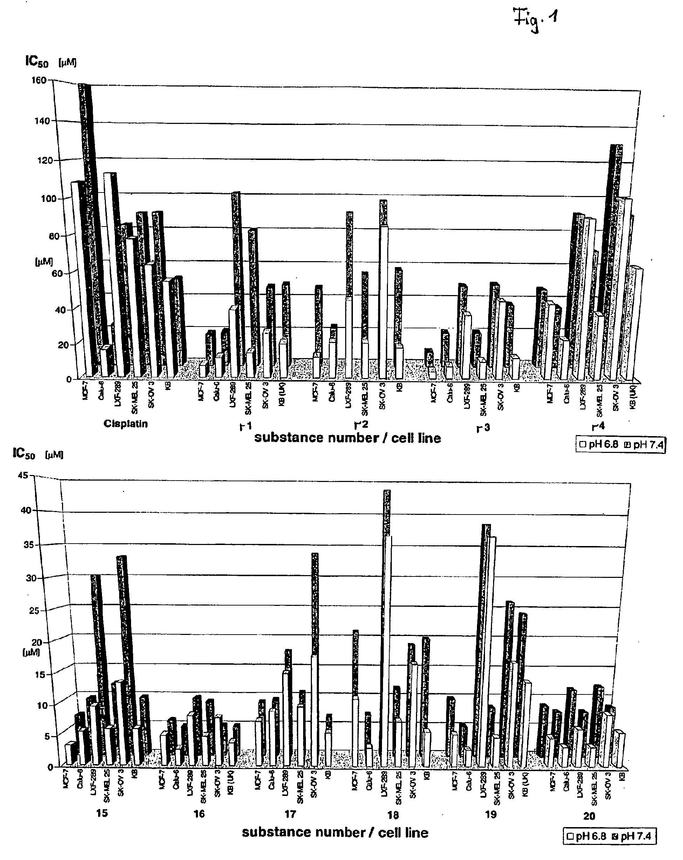

Secondary alkyl systems **15-20** displayed the highest activity of all compounds studied. When compared with the corresponding n-alkyl systems two to three fold reduced IC₅₀ values were obtained. This effect is most obvious by comparing **r4** and **20.** The secondary cyclohexyl derivative is approximately 10-fold more cytotoxic on tumor cells than the corresponding n-hexyl derivative (c.f. Table 1 and Fig. 1).

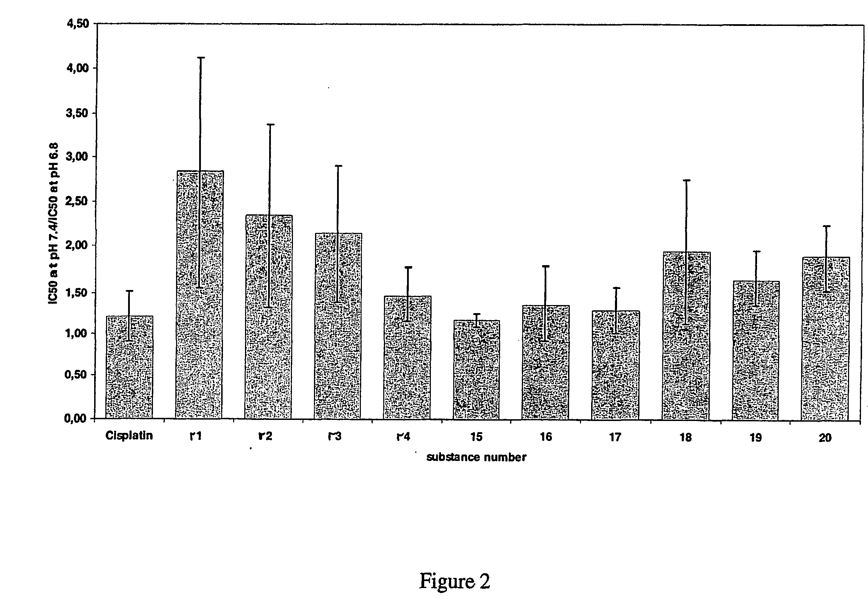

Furthermore, it has been found out that for all tested bis(O-alkyl-dithiocarbonato)platinum compounds the IC₅₀ values at pH 6.8 have been lower than those at pH 7.4. Within the tested compounds **15, 16** and **20** share almost equal cytotoxicity although there is a significant difference in the pH 6.8/7.4 activity quotient (Fig. 2). While this values was 2.18 ± 0.49 for **15** it was somewhat lower (1.88 ± 0.36) for **20** (being the most active compound) and as low as 1.35 ± 0.43 for **16.** The difference between **15** and **16** is significant at p = 0.033 (T-test).

**Table 1**

| Cell line | | IC₅₀ ± SD^{a} [µM] | | | | | | | | | | | |
|---|---|---|---|---|---|---|---|---|---|---|---|---|---|
| | | **MCF-7** | | **Calu-6** | | **LXF-289** | | **SK-MEL-25** | | **SK-OV-3** | | **KB** | |
| Compound | structure | pH 6.8 | pH 7.4 | pH 6.8 | pH 7.4 | pH 6.8 | pH 7.4 | pH 6.8 | pH 7.4 | pH 6.8 | pH 7.4 | pH 6.8 | pH 7.4 |
| Cisplatin | (NH₃)₂PtCl₂ | 108 ± 27 | 160 ± 30 | 16 ± 1.0 | 25 ± 5.7 | 113 ± 22.3 | 83 ± 23.0 | 78 ± 3.3 | 90 ± 25.0 | 64 ± 0.7 | 90 ± 27.7 | 55 ± 2.0 | 52 ± 3.3 |
| Bis(O-methyldithiocarbonato)platinum (II) **(r1)** | 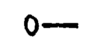 | 7±1.0 | 19±4.4 | 11±2.9 | 21±5.6 | 39±9.5 | 102±19.3 | 14±5.0 | 80±25.9 | 26±2.4 | 47±2.9 | 20±1.0 | 49±2.0 |
| Bis(O-ethyldithiocarbonato)platinum(II) **(r2; thioplatin)** | 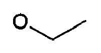 | 12 ± 0.2 | 47 ± 5.3 | 21 ± 3.9 | 24 ± 0.9 | 45 ± 8.0 | 91 ± 24.9 | 21 ± 3.0 | 56 ± 6.2 | 86 ± 3.2 | 98 ± 16.5 | 18 ± 4.0 | 58±5.0 |
| Bis(O-propyldithiocarbonato)platinum(II) **(r3)** | 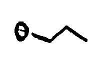 | 4±1.0 | 9±3.0 | 7±1.1 | 21± 0.9 | 37±9.0 | 49±5.0 | 10±1.5 | 21±1.9 | 45±27.0 | 50±1.0 | 12±1.0 | 38±3.0 |
| Bis(O-hexyldithiocarbonato)platinum(II) **(r4)** | 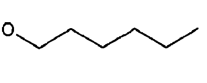 | 44 ± 5.5 | 47 ± 25.5 | 23 ± 7.3 | 37 ± 2.7 | >91 | >91 | 37 ± 3.3 | 70 ± 10.2 | 102 ± 13.0 | 130 ± 7.0 | 64 ± 4.0 | >91 |
| Bis(O-isopropyldithiocarbonato)platinum(II) **(15)** | 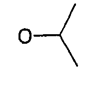 | 3 ± 0.9 | 7±3.0 | 6 ± 0.6 | 10 ± 0.6 | 10 ± 1.7 | 30 ± 0.4 | 6 ± 0.4 | 12 ± 1.5 | 14 ± 2.1 | 33±8.0 | 6 ±0.4 | 10 ±0.9 |
| Bis(O-(1-ethyl)propyldithiocarbonato)platinum(II) **(16)** | 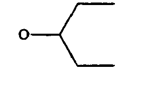 | 5 ± 0.2 | 6 ± 1.5 | 3 ± 0.4 | 5 ± 0.4 | 8 ± 1.3 | 10 ± 0.8 | 5 ± 0.2 | 9 ± 0.4 | 8 ± 1.5 | 5 ± 0.6 | 4 ± 0.6 | 5 ± 0.4 |
| Bis(O-(1,2,2-trimethyl)propyldithiocarbonato)platinum(II) **(17)** | 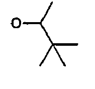 | 7 ± 0.5 | 9 ± 0.5 | 9 ± 0.2 | 9 ± 0.7 | 15 ± 2.7 | 18 ± 2.5 | 10 ± 0.2 | 11 ± 0.2 | 18 ± 0.9 | 34 ± 1.5 | 5 ± 0.5 | 7 ± 1.3 |
| Bis(O-cyclobutyldithiocarbonato)platinum(II) **(18)** | 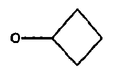 | 11 ± 0.8 | 21 ± 1.0 | 3 ± 0.4 | 7 ± 1.2 | 37 ± 1.4 | 44 ± 0.8 | 7 ± 1.0 | 12 ± 3.7 | 17 ± 2.9 | 19 ± 4.1 | 6 ± 0.6 | 20 ± 2.9 |
| Bis(O-cyclopentyldithiocarbonato)pfatinum(II) **(19)** | 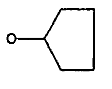 | 5 ± 0.4 | 10 ± 0.4 | 3 ± 0.2 | 5 ± 0.2 | 37 ± 0.6 | 38 ± 1.0 | 5 ± 0.6 | 9 ± 1.7 | 17 ± 1.5 | 26 ± 6.8 | 14 ± 2.9 | 24 ± 3.7 |
| Bis(O-cyclohexyldithiocarbonato)platinum(II) **(20)** | 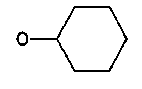 | 5 ± 0.2 | 9 ± 1.5 | 3 ± 0.4 | 8 ± 2.0 | 6 ± 1.8 | 12 ± 1.1 | 3 ± 0.7 | 8 ± 2.4 | 9 ± 2.2 | 12 ± 1.6 | 6 ± 0.2 | 9 ± 0.7 |

| | | | | | | | | | | | | | |
|---|---|---|---|---|---|---|---|---|---|---|---|---|---|
| ^{a}IC₅₀ -values were determined from dose response curves at pH 6.8 and pH 7.4, respectively. Values are the mean of four separate assays ± SD. r1 to r4 are reference compounds | | | | | | | | | | | | | |

## Claims

1. A pharmaceutical composition containing at least one of the following platinum complex compounds:
Bis(O-isopropyl-dithiocarbonato)platinum(II), Bis(O-(1-ethyl)propyl-dithiocarbonato)platinum(II), Bis(O-(1,2,2-trimethyl)propyl-dithiocarbonato)platinum(II), Bis(O-cyclobutyl-dithiocarbonato)platinum(II), Bis(O-cyclopentyldithiocarbonato)platinum(II) and/or Bis(O-cyclohexyldithiocarbonato)platinum(II).

2. The pharmaceutical composition according to claim 1, comprising additionally one or more of the following compounds: cyclosporine, rapamycin, 15-desoxyspergualin, OKT3 and/or azothioprine.

3. The pharmaceutical composition according to claim 1 or 2, comprising additionally a cytokine, interferon and/or a further cytostatic agent.

4. The pharmaceutical composition according to claim 3, wherein the additional cytostatic agent is methotrexate, aminopterin, dacarbacine, nitroso urea compounds, fluorouracil, bleomycin, daunomycin, daunorubicin, doxorubicin, mithramycin, mitomycin C.

5. Use of at least one platinum complex compound for preparing a pharmaceutical composition for treating a cancerous or an autoimmune disease, wherein said platinum complex compound is selected from the following compounds:
Bis(O-isopropyl-dithiocarbonato)platinum(II), Bis(O-(1-ethyl)propyl-dithiocarbonato)platinum(II), Bis(O-(1,2,2-trimethyl)propyl-dithiocarbonato)platinum(II), Bis(O-cyclobutyl-dithiocarbonato)platinum(II), Bis(O-cyclopentyldithiocarbonato)platinum(II) and/or Bis(O-cyclohexyldithiocarbonato)platinum(II).

6. Use according to claim 5, wherein the cancerous disease is selected from testicular tumors, ovarian carcinomas, bladder carcinomas, colon carcinomas, prostatic carcinomas, parvocellular and non-parvocellular bronchial carcinomas, carcinomas of the cephalic and cervical parts, carcinomas of the thoracic and abdominal regions, cervical and endometrial carcinomas, sarcomas, melanomas and leukemias.

7. A process for the production of a pharmaceutical composition according to any one of claims 1 to 4, **characterized in that** a compound as defined in claim 1 and, optionally, one or more of the compounds as defined in any one of claims 2 to 4 are mixed with a pharmaceutically compatible carrier or diluent.

## Patentansprüche

1. Pharmazeutische Zusammensetzung, enthaltend mindestens eine der folgenden Platinkomplexverbindungen:
Bis(O-isopropyl-dithiocarbonato)platin(II), Bis(O-(1-ethyl)propyldithiocarbonato)platin(II), Bis(O-(1,2,2-trimethyl)propyl-dithiocarbonato)platin(II), Bis(O-cyclobutyl-dithiocarbonato)platin(II), Bis-(O-cyclopentyl-dithiocarbonato)platin(II) und/oder Bis(O-cyclohexyldithiocarbonato)platin(II).

2. Pharmazeutische Zusammensetzung nach Anspruch 1, zusätzlich umfassend eine oder mehrere der folgenden Verbindungen: Cyclosporin, Rapamycin, 15-Desoxyspergualin, OKT3 und/oder Azothioprin.

3. Pharmazeutische Zusammensetzung nach Anspruch 1 oder 2, zusätzlich umfassend ein Zytokin, Interferon und/oder ein weiteres zytostatisches Mittel.

4. Pharmazeutische Zusammensetzung nach Anspruch 3, wobei das zusätzliche zytostatische Mittel Methotrexat, Aminopterin, Dacarbacin, Nitrosoharnstoffverbindungen, Fluorouracil, Bleomycin, Daunomycin, Daunorubicin, Doxorubicin, Mithramycin, Mitomycin C ist.

5. Verwendung von mindestens einer Piatinkompjexverbindung zur Herstellung einer pharmazeutischen Zusammensetzung zur Behandlung einer Krebserkrankung oder einer Autoimmunerkrankung,
wobei die Platinkomplexverbindung aus einer der folgenden Verbindungen ausgewählt ist: Bis(O-isopropyl-dithiocarbonato)platin(II),
Bis(O-1(1-ethyl)propyl-dithiocarbonato)platin(II), Bis(O-(1,2,2-trimethyl)propyl-dithiocarbonato)platin(II), Bis(O-cyclobutyl-dithiocarbonato)platin(II), Bis(O-cyclopentyl-dithiocarbonato)platin(II) und/oder Bis(O-cyclohexyl-dithiocarbonato)platin(II).

6. Verwendung nach Anspruch 5, wobei die Krebserkrankung aus Hodentumoren, Ovarialkarzinomen, Blasenkarzinomen, Kolonkarzinomen, Prostatakarzinomen, kleinzelligen und nicht kleinzelligen Bronchialkarzinomen, Karzinomen der kephalischen and zervikalen Teile, Karzinomen der thorakalen und abdominalen Bereiche, zervikale und endometrialen Karzinomen, Sarkomen, Melanomen und Leukämien ausgewählt ist.

7. Verfähren zur Herstellung einer pharmazeutischen Zusammensetzung nach einem der Ansprüche 1 bis 4, **dadurch gekennzeichnet, dass** eine im Anspruch 1 definierte Verbindung und wahlweise eine oder mehrere der in einem der Ansprüche 2 bis 4 definierten Verbindungen mit einem pharmazeutisch annehmbaren Träger oder Verdünnungsmittel gemischt sind.

## Revendications

1. Composition pharmaceutique comprenant au moins un des composés de complexes de platine suivants : un Bis(O-isopropyle-dithiocarbonato)platine(II), un Bis(O-(1-éthyle)propyle-dithiocarbonato)platine(II), un Bis(O-(1,2,2-triméthyle)propyle-dithiocarbonato)platine(II), un Bis(O-cyclobutyledithiocarbonato)platine(II), un Bis(O-cyclopentyle-dithiocarbonato)platine(II) et/ou un Bis(O-cyclohéxyle-dithiocarbonato)platine(II).

2. Composition pharmaceutique selon la revendication 1, comprenant en outre un ou plusieurs des composés suivants : une cyclosporine, une rapamycine, une 15-désoxyspergualine, un OKT3 et/ou une azothioprine.

3. Composition pharmaceutique selon la revendication 1 ou 2, comprenant en outre une cytokine, un interféron et/ou un autre agent cytostatique.

4. Composition pharmaceutique selon la revendication 3, dans laquelle l'agent cytostatique additionnel est un méthotréxate, une aminoptérine, une dacarbacine, des composés de nitroso urée, une fluorouracile, une bléomycine, une daunomycine, une daunorubicine, une doxorubicine, une mithramycine, une mitomycine C.

5. Utilisation d'au moins un composé de complexe de platine pour la préparation d'une composition pharmaceutique pour traiter une maladie cancéreuse ou autoimmune, dans laquelle ledit composé de complexe de platine est sélectionné parmi les composés suivants : un Bis(O-isopropyle-dithiocarbonato)platine(II), un Bis(O-(1-éthyle)propyle-dithiocarbonato)platine(II), un Bis(O-(1,2,2-triméthyle)propyledithiocarbonato)platine(II), un Bis(O-cyclobutyle-dithiocarbonato)platine(Il), un Bis(O-cyclopentyle-dithiocarbonato)platine(II) et/ou un Bis(O-cyclohéxyledithiocarbonato)platine(II).

6. Utilisation selon la revendication 5, pour laquelle la maladie cancéreuse est sélectionnée parmi des tumeurs testiculaires, des carcinomes ovariens, des carcinomes de la vessie, des carcinomes du côlon, des carcinomes prostatiques, des carcinomes bronchiques parvocellulaires et non parvocellulaires, des carcinomes des parties céphaliques et cervicales, des carcinomes des régions thoraciques et abdominales, des carcinomes cervicaux et endométriaux, des sarcomes, des mélanomes et des leucémies.

7. Procédé de production d'une composition pharmaceutique selon l'une quelconque des revendications 1 à 4, **caractérisé en ce qu'**un composé, tel que défini dans la revendication 1, et facultativement un ou plusieurs des composés, tels que définis dans l'une quelconque des revendications 2 à 4, sont mélangés avec un porteur ou un diluant pharmaceutiquement compatible.
